# EUROPEAN PATENT APPLICATION

(11) **EP 2 859 882 A1**
(43) Date of publication of application: **15.04.2015**
(21) Application number: 13188145.0
(22) Date of filing: 10.10.2013
(51) Int. Cl.: A61K 8/55, A61Q 19/08, A61K 8/92

(54) **Natural skin care composition**

(71) Applicant: Aulive NV, 8902 Ieper (BE)
(72) Inventor: Geryl, Patrick, 2100 Deurne (BE)
(74) Representative: De Clercq, Ann G. Y.

(57) **Abstract**

The present invention relates to natural skin care compositions comprising coconut oil and lecithin at a concentration of at least 2.5 % by total weight of the composition. The invention also provides for their use for prophylactic or remedial treatment of a skin exhibiting reduced elasticity, and methods for producing these compositions.

## Description

### FIELD OF THE INVENTION

This invention relates to all-natural formulations for topical skin care compositions.

### BACKGROUND OF THE INVENTION

Reduced skin elasticity, reduced firmness, increased sagginess, damage caused by ultraviolet radiation, development of fine lines and wrinkles, development of age spots, dry skin, etcetera, are considered visually undesirable.

Until now the cosmetic industry has tried to reverse these undesirable effects in skin predominantly by lubrication of topical compositions containing soothing agents as exemplified by commercial hand lotion products and the like.

Recently attention has been directed to agents which address the underlying processes involved in skin damage. Most of the recent studies concentrate on one agent at the time, and not to the synergistic effects of several agents combined in one skin care composition. Furthermore, most of the products against these effects known today contain mainly synthetic ingredients, with only a very small amount of natural ingredients. Due to high number of synthetic ingredients used in the skin care products, human blood and lipid tissues may contain up to 400 hazard chemicals originating from the cosmetics and food products, according to the U.S. environmental protection agency (EPA). Those chemicals may irritate sensitive skin and be hazardous for health and bring about various cancers and various chronic disorders. Therefore, there is a need for the development of "all-natural" cosmetic compositions.

### SUMMARY OF THE INVENTION

The present invention relates to a skin care composition comprising coconut oil and lecithin, wherein said composition comprises lecithin at a concentration of at least 2.5 % by total weight of the composition, preferably at least 5%, 6%, 7%, 8%, 9%, 10%, 15% or 20%.

In preferred embodiments, said composition is free of synthetic additives and preservatives.

In preferred embodiments, the skin care composition comprises sunflower lecithin, liquid soy lecithin, or a mixture thereof.

In embodiments, the lecithin is liquid lecithin, preferably liquid sunflower lecithin and/or liquid soy lecithin, more preferably liquid sunflower lecithin.

In further embodiments, the skin care composition further comprises red palm oil.

In further embodiments, the skin care composition further comprises a natural source of vitamin C, wherein said natural source of vitamin C is preferably selected from the group consisting of a plant, a herb, a leave, a vegetable, a fruit and any mixture thereof, more preferably selected from the group consisting of Kakadu Plum, Amazonian Camu Camu Berry, Sea Buckthorn, Rose Hip and mixtures thereof.

In further embodiments, the skin care composition further comprises a natural source of vitamin A, a natural source of vitamin B, a natural source of tocotrienol, a natural source of vitamin E, or any mixture thereof.

In preferred embodiments, the skin care composition comprises coconut oil at a concentration from 25% to 85% by weight of the composition.

In preferred embodiments, the skin care composition comprises lecithin at a concentration from 2.5% to 50% by weight of the composition, more preferably from 5% to 50%, from 10% to 50%, from 15% to 50% or from 20% to 50%, even more preferably from 11 % to 35%, from 15% to 35%, from 20% to 35%, and yet more preferably from 20% to 30% or from 20% to 25%.

In preferred embodiments, the skin care composition comprises red palm oil at a concentration from 2.5% to 30% by weight of the composition, preferably from 7.5% to 20% and more preferably from 10 % to 15%.

In embodiments, the skin care composition as defined above is a cream, preferably a face cream, body cream, or hand cream.

In further embodiments, the skin cream further comprises white tea, preferably white matcha *(Thea sinensis* or varieties).

In further embodiments, the skin cream comprises coconut oil at a concentration from 50% to 70% by weight of the composition, preferably from 60% to 67%.

In other embodiments, the skin care composition as defined above is a skin or face mask.

In further embodiments, the skin or face mask further comprises green tea, preferably green matcha. *(Thea sinensis* or varieties).

In further embodiments, the skin or face mask comprises coconut oil at a concentration from 25% to 40% by weight of the composition, preferably from 30% to 35%.

In further embodiments, the skin or face mask further comprises clay, preferably montmorillonite, or volcanic ash.

Furthermore, the present invention encompasses the use of a skin care composition according to the present invention in a prophylactic or remedial treatment of a skin exhibiting reduced elasticity by topical application of said composition to the skin.

A further aspect relates to a method for preparing a skin care composition disclosed herein involving mixing the different components of said composition.

### DETAILED DESCRIPTION OF THE INVENTION

Before the present invention is described in detail, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

When describing the compositions and processes of the invention, the terms used are to be construed in accordance with the following definitions, unless a context dictates otherwise.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all numbers and fractions subsumed within the respective ranges, as well as the recited endpoints.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

According to a first aspect, the present invention provides a skin care composition comprising coconut oil and lecithin, wherein said composition comprises lecithin at a concentration of at least 2.5 % by total weight of the composition.

A skin care composition according to the present invention can be a cream, such as, e.g., a face cream, a body cream, or a hand cream, or a mask, such as, e.g., a face mask or a skin mask. Preferably the skin care composition is a cream, more preferably a face cream or a hand cream.

The composition according to the present invention can hydrate the skin, increase the firmness of the skin, reduce the appearance of fine lines or wrinkles on the skin, reduce the appearance of age spots on the skin, and/or provide mild sun protection. The specific combination of the selected natural ingredients in the skin care composition according to the present invention provides a synergistic effect for the prevention and/or treatment of skin exhibiting reduced elasticity.

Furthermore, the skin care composition according to the present invention can be used without synthetic additives and preservatives. Such composition is free of the toxicity associated with many of the synthetic preservatives and other chemicals in use today. This is particularly advantageous for consumers sensitive to chemicals. In addition, due to the absence of synthetic ingredients, the composition of the invention is edible as all components are edible.

In preferred embodiments, the composition is free of synthetic additives and preservatives.

In particularly preferred embodiments, the composition is "all-natural" wherein each of the components contained is obtained from a natural source. The natural source can be, e.g., plant, fruit, vegetable, leaves, herbs, minerals and mixtures thereof.

The natural components can be used in the form in which they exist in their natural source or in a substantially unmodified form (i.e. only requiring minimal processing), or they can first undergo substantive processing prior to use. For example, coconut oil as contemplated herein includes "virgin", "pure" or "unrefined" coconut oil, which is the coconut oil extracted from fresh coconut meat by cold pressing, as well as "refined" coconut oil (e.g. Refined, Bleached and Deodorized (RBD) coconut oil), which is extracted from dried coconut meat and has undergone substantive processing such as, e.g., bleaching, deodorizing, hydrogenation, etc. and/or any combination thereof.

The natural ingredients are preferably used in a substantially unmodified form, namely in the form which exists in the natural origin.

In further embodiments, the composition is organic.

With the term "organic" is meant herein that each of the components is organically produced. Organic components must be free of artificial additives, and are typically processed with fewer artificial methods, materials and conditions. If crops are involved, this encompasses that the crops are not genetically modified (i.e. non-GMO crops) and that they are grown organically, i.e. without the use of synthetic pesticides or other chemical plant protection agents.

Coconut oil is an edible oil extracted from the kernel or meat of matured coconuts harvested from the coconut palm (*Cocos nucifera*). Coconut oil can be extracted from fresh meat (e.g. (extra) virgin coconut oil) or dried meat or copra (e.g. refined coconut oil). Coconut oil can be extracted using expeller-pressed or cold-pressed technology.

"Expeller pressing" is a chemical-free mechanical process that extracts oil from seeds and nuts. The temperature reached during pressing depends on the hardness of the nut or seed. The harder the nut or seed, the more pressure required to extract the oil, which in turn creates more friction and higher heat. Oils that are "cold pressed" are expeller pressed in a heat-controlled environment to keep temperatures below 49°C. In this sense, it is more beneficial to choose a cold pressed oil, as the more heat that is applied, the more the nutritional benefits are normally compromised and the more the quality of the oil is degraded.

In embodiments, the composition comprises virgin coconut oil, more preferably extra virgin coconut oil. Virgin coconut oil and extra virgin coconut oil as used herein are obtained through cold pressing. In further embodiments, the composition comprises organic virgin coconut oil, more preferably organic extra virgin coconut oil.

Coconut oil is natural oil that can be used as a natural preservative. It contains ingredients that have anti-microbial properties, which help keep skin care formulas bug-free without harsh chemical preservatives. These natural properties kill bacteria on the skin, too, which helps to reduce acne breakouts. Due to the anti-microbial properties of coconut oil, the skin care composition of the present invention is free of synthetic additives and preservatives.

Coconut oil has a unique combination of fatty acids; lauric acid, capric acid, caprylic acid, myristic acid and palmitic acid, and therefore it has a way of penetrating skin that few other oils have.

Coconut oil is also rich in natural antioxidants. That means that the natural antioxidants are also going to help protect the skin from free radical damage, preserving a more youthful look. These antioxidants are also reputed to help reduce the redness and limit the damage of sun exposure.

Studies have shown that when dry, rough and scaly skin is treated with coconut oil, skin shows significant improvement in hydration, but also an increase in the lipid levels found on the surface of the skin.

A skin care composition according to the present invention preferably comprises coconut oil at a concentration from 25% to 85% by total weight of the composition.

The concentration of coconut oil may vary depending on the formulation of the skin care composition. Creams typically comprise higher concentrations than masks.

A skin care cream according to the present invention preferably comprises coconut oil at a concentration from 50% to 70% and more preferably from 60% to 67%.

A skin care mask according to the present invention preferably comprises coconut oil at a concentration from 25% to 40% and more preferably from 30% to 35%.

"Lecithin" is a generic term to designate any group of yellow-brownish fatty substances occurring in animal and plant tissues composed of phosphoric acid, choline, fatty acids, glycerol, glycolipids, triglycerides, and phospholipids e.g., phosphatidylcholine, phosphatidylethanolamine and phosphatidylinositol.

Lecithin can be used as a source of phospholipids in the compositions according to the invention. "Phospholipids" are a class of lipids that are a major component of all cell membranes as they can form lipid bilayers. Phospholipids share a high structural similarity with skin lipids and thus have many advantages such as strong tissue affinity, biodegradability and very low toxicity. Phospholipids are widely found in all animals and plants in nature and are non-irritant, non-comedogenic and non-allergenic. The unique affinity of membrane lipids to the skin has the ability to penetrate the epidermis and enhance the bioavailability of other active ingredients to carry substances to the right cell level. Phospholipids have film-forming properties and long-lasting effects on skin moisturization. Phospholipids help increase hydration levels in the skin without modifying the water loss rate of the skin.

Liposomes are formed when a group of phospholipids are placed on the skin; they spontaneously arrange themselves to match their water loving heads together and their oil loving tails together to form a microscopic sphere. These spheres are similar in construction to an actual cell membrane. Lecithin can also form liposomes.

Lecithin can be used in the present invention in any available form, such as, for example, in powder form, as granules, as liquid, as capsules, etc.

Liquid lecithin is preferably used in the present invention. Liquid lecithin improves processability of solid coconut oil (melting point around 24 °C). Furthermore, it provides softer texture for the composition and improves topological application of the composition to the skin. Suitable liquid lecithins for use in the present invention are for example liquid sunflower lecithin, liquid soy lecithin and mixtures thereof. Accordingly, in preferred embodiments, the composition comprises liquid lecithin.

In embodiments, the composition comprises sunflower lecithin and/or soy lecithin, preferably sunflower lecithin.

Sunflower lecithin is extracted from sunflower oil. Soy lecithin is extracted from soybean oil. Preferably, the oils are cold-pressed from the seeds and subsequently the lecithin is isolated through separation of the oil.

Soy lecithin and sunflower lecithin comprise various phospholipids e.g. phosphatidylcholine (PC), phosphatidylethanolamine (PE), Phosphatidic Acid (PA) and phosphotidylinositol (PI).

PC is considered the most important phospholipid conferring the best dispersing and emulsifying properties of lecithin. PC is also called vitamin F. PC has a high percentage of unsaturated fatty acid chains, 70% of linoleic acid and 6% of linolenic acid. Vitamin F has been found to be crucial for fat metabolism and for the release of fat from fatty tissues and to restore an important barrier function of the skin. A recent study concluded that the water loss from skin is inversely proportional to the linoleic acid content of the skin.

Soy lecithin and sunflower lecithin comprise also various fatty acids e.g. C16 Palmitic acid, C18 Stearic Acid, C18:1 Oleic Acid, C18:2 Linoleic Acid and C18:3 Linolenic Acid.

In further embodiments, the composition comprises organic lecithin, preferably organic sunflower lecithin and/or organic soy lecithin, more preferably organic sunflower lecithin.

A non-limiting example of organic sunflower lecithin is LECICO Sun 400 ORGANIC provided by Lecico Gmbh. The use of (organic) sunflower lecithin has the advantage that it is not allergenic. Furthermore, sunflower seeds have, as of yet, not been genetically modified, hence, there is no risk for contamination by genetically modified seeds.

A skin care composition according to the present invention preferentially comprises at least 2.5% by weight of the composition of lecithin, more preferably at least about 5%, 6%, 7%, 8%, 9% 10%, 15% or 20%. Preferably the skin care composition comprises lecithin at a concentration from 2.5% to 50% by weight of the composition, more preferably from 5% to 50%, from 10% to 50%, from 15% to 50% or from 20% to 50%, even more preferably from 11 % to 35%, from 15% to 35%, or from 20% to 35%, and yet more preferably from 20% to 25%.

The skin care composition according to the present invention may further comprise red palm oil.

Red palm oil is derived from the mesocarp of the fruit of the oil palm. Red palm oil is naturally reddish in color because of its high beta-carotene content.

In preferred embodiments, the composition comprises virgin red palm oil, including extra virgin red palm oil, which is extracted from fresh, not-dried, fruit through cold pressing.

In further embodiments, the composition comprises organic red palm oil, preferably organic virgin red palm oil or organic extra virgin red palm oil.

Red palm oil is rich in vitamin E and especially rich in alpha-tocotrienol which is chemical compound considered to be vitamin E. Alpha-tocotrienol has been shown to be 40 - 60 times more potent than alpha-tocopherol as an antioxidant in the prevention of lipid peroxidation. Tocotrienols topically applied onto the skin was found to penetrate rapidly through the skin and the highest concentrations are found in the uppermost 5 microns. Tocotrienol-treated skin contained Vitamin E at a concentration 7-30 fold higher than normal values.

Red palm oil is also rich source of vitamin A. Red palm oil has 15 times more Retinol Equivalents than carrot and 300 times more than tomato. Vitamin A is measured in retinol equivalents (RE) which allows the different forms of vitamin A to be compared.

Additionally, red palm oil is rich source of carotenes. Carotenes are the precursors of vitamin A, with β-carotene having the highest provitamin A activity.

A skin care composition according to present invention preferentially comprises red palm oil at a concentration from 2.5% to 30% by weight of the composition, preferably from 7.5% to 20% and more preferably 10% to 15%.

The skin care composition according to the present invention may further comprise a natural source of vitamin C.

Vitamin C stimulates collagen production in a skin, improves elasticity, and works along with skin-softening vitamin E to protect skin from environmental aggressors, which in turn holds the skin together for longer. Collagen is one of the main structural proteins in human dermis, generally collagen is the protein in charge of the structure of the skin. The lack and atrophy of collagen induces the appearance of wrinkles and beginning of aging. Vitamin C has effects on skin whitening and anti-oxidation, which helps maintain skin healthy. It brightens the skin, reduces fine lines and wrinkles and generally makes the skin more youthful overall.

Vitamin C used in the present invention is obtained from a natural source. The natural source of vitamin C can be selected from the group consisting of a plant, a herb, a leave, a vegetable or a fruit and mixtures thereof. Preferably, the natural source of vitamin C can be selected from the group consisting of Kakadu Plum, Amazonian Camu Camu Berry, Sea Buckthorn, Rose Hip and mixtures thereof.

Green tea (derived from *Thea sinensis* or its cultivation varieties), preferably green matcha and/or white tea (derived from *Thea sinensis* or its cultivation varieties), preferably white matcha, may be added to the compositions according to the present invention. White tea is preferably added to skin care creams as taught herein, whereas green tea is preferably added to skin care masks as taught herein. Green tea and white tea are a source of catechins.

The main catechins in green tea include gallocatechin (GC), epigallocatechin (EGC), epicatechin (EC), and epigallocatechin gallate (EGCG). Catechins are prominent compounds in green tea extract and represent intensive antioxidant activity. In vitro studies have indicated that catechins prevent skin damage by decreasing collagen degradation and improving the extracellular matrix structure to tolerate UV radiation and pollution. The physicochemical properties of the catechin molecule sets limitations to their skin permeation. This problem can be overcome by formulating a composition comprising phospholipids, which improve catechins skin permeability.

White tea is also rich source of catechins. White tea has shown potential advantages as a skin rejuvenation agent. Recent study has shown that white tea has potent antioxidant activity and the ability to inhibit matrix metalloproteinases (MMP). MMP are enzymes that degrade the key proteins of skin matrix, particularly collagen and elastin, leading to wrinkles and loss of firmness. A certain level of MMP in the skin is healthy and necessary but excess MMP activity contributes to skin aging.

A skin care composition, in particular a skin cream, according to the invention preferably comprises white tea, preferably white matcha, at a concentration from 1% to 5% by weight of the composition.

The skin care composition according to the present invention may further comprise one or more further optional ingredients.

Suitable optional ingredients are for example natural vitamins and minerals from plants, fruits, vegetables, leaves and herbs extracts.

In embodiments, the skin care composition according to the invention further comprises a natural source of vitamin E, a natural source of tocotrienol, a natural source of vitamin A, and/or a natural source of vitamin B.

In embodiments, the skin care composition according to the invention further comprises Shea butter, cocoa butter, and/or Agran oil.

Clay may also be added to the compositions of the present invention.

Clay is rich in minerals and active enzymes. Clay treatments stimulate the circulation of blood and lymph, remove dead skin cells and absorb dirt and other surface fats. They also tone and strengthen the connective tissues. Clay stimulates blood circulation and draws toxins and superfluous fat from the skin. It contracts and tones the skin and strengthens the connective tissue. Clay is known in use for dirty and flaccid skin, acne, for poor blood circulation and to soothe eczema and sunburn.

A preferred clay that can be used in the composition according to the invention is montmorillonite.

Volcanic ash may also be added to the compositions of the present invention.

Volcanic ash exerts powerful cleansing and purifying qualities for the skin. Volcanic ash is rich in minerals essential for metabolism. Minerals are including for example calcium, potassium, sodium, magnesium. Volcanic ash exfoliates old scales mildly and inhibits bacteria naturally.

In an embodiment, the skin care composition comprises coconut oil and sunflower lecithin, wherein said composition comprises lecithin at a concentration of at least 2.5 % by total weight of the composition.

In one embodiment a skin care composition according to the present invention comprises coconut oil from 25% to 80% by weight of the composition, and lecithin, preferably sunflower lecithin and/or liquid soy lecithin, more preferably sunflower lecithin, from 2.5% to 50% by weight of the composition and red palm oil from 2.5% to 30% by weight of the composition.

In a further embodiment, the skin care composition is a skin care cream, said composition comprising coconut oil, preferably (extra) virgin coconut oil, lecithin, preferably sunflower lecithin and/or liquid soy lecithin, more preferably sunflower lecithin, and red palm oil, preferably (extra) virgin red palm oil, mixed with Rose Hip and white matcha (*Thea sinensis)*.

In a further embodiment, wherein the skin care composition is either a face mask or a body mask, said composition comprises coconut oil, preferably (extra) virgin coconut oil, lecithin, preferably sunflower lecithin and/or liquid soy lecithin, more preferably sunflower lecithin and red palm oil, preferably (extra) virgin red palm oil, mixed with clay, preferably montmorillonite, and green tea, preferably green matcha (*Thea sinensis)*.

According to the second aspect, the present invention provides for the use of a skin care composition according to present invention in a prophylactic or remedial treatment of a skin exhibiting reduced elasticity by topical application of said composition to the skin.

In a related aspect, the invention provides a skin care composition according to the present invention for use in a prophylactic or remedial treatment of a skin exhibiting reduced elasticity by topical application of said composition to the skin.

Also disclosed herein is a method for a prophylactic or remedial treatment of a skin exhibiting reduced elasticity comprising the topical application of a skin care composition according to the present invention to the skin.

A topical application of a composition according to the present invention to the skin can be in the décolleté region, facial skin, hands, legs and/or the whole skin of the body.

Yet another aspect of the present invention relates to a method for preparing a skin care composition disclosed herein involving mixing the different components of said composition.

A skin cream according to the present invention can be made by mixing coconut oil and lecithin with White Matcha to 40 degrees. After a day the mixture is reheated. This process can be repeated. This way the catechins can move into the mixture. Optionally, red palm oil is added subsequently into the mixture and mixed. All further optional ingredients are added subsequently and mixed. The mixture is finally filtered or centrifuged to obtain a smooth texture (i.e. without particles).

A face mask and/or skin mask according to the present invention can be made by mixing the coconut oil with the lecithin, and the green tea at 40 degrees Celsius. Subsequently the mixture is allowed to cool to 30 °C. After the cooling, Red Palm Oil and natural source of vitamin C are optionally added and mixed. At last either the active clay or volcanic ash is added and mixed. No metal may be used because this deactivates the active clay or volcanic ash. So the mixer is preferably made of wood or glass. Plastic is a last option, because chemicals from the plastic can dissolve in the formulation. This is extremely important and makes the mixture difficult to make. For skin masks, there is no need for a filtration or centrifugation step. Also the product has to be stored in a dark glass container, un-penetrable by UV light.

### EXAMPLES

### Example 1: Face or skin cream according to the present invention

| **Ingredient** | **Quantity** |
|---|---|
| Organic Extra Virgin Coconut oil | 66.6 % |
| Organic Liquid Sunflower Lecithin (LECICO Sun 400 ORGANIC) | 22.2 % |
| Organic Extra Virgin Red Palm Oil | 11.1% |

### Example 2: Face or skin cream according to the present invention

| **Ingredient** | **Quantity** |
|---|---|
| Organic Extra Virgin Coconut oil | 60% |
| Organic Liquid Sunflower Lecithin (LECICO Sun 400 ORGANIC) | 22 % |
| Organic Extra Virgin Red Palm Oil | 11 % |
| Organic Rose Hip | 3.5% |
| Organic White Matcha *Thea sinensis* | 3.5% |

### Example 3: Face or skin-mask according to the present invention:

| **Ingredient** | **Quantity** |
|---|---|
| Organic Virgin Coconut oil | 33% |
| Organic Liquid Sunflower Lecithin (LECICO Sun 400 ORGANIC) | 20% |
| Organic Virgin Red Palm Oil | 11% |
| Rose Hip | 8% |
| active clay (montmorillonite) | 20% |
| Green Matcha | 8% |

## Claims

1. A skin care composition comprising coconut oil and lecithin, wherein said composition comprises lecithin at a concentration of at least 2.5 % by total weight of the composition, preferably from 2.5 % to 50%.

2. The skin care composition according to claim 1, wherein said lecithin is liquid soy lecithin, sunflower lecithin, or a combination thereof.

3. The skin care composition according to any one of the preceding claims 1 to 2, wherein said composition comprises lecithin from 5 to 50 % by weight of the composition, preferably from 11% to 35%, and more preferably from 20% to 25%.

4. The skin care composition according to any one of the preceding claims 1 to 3, wherein said composition further comprises red palm oil, preferably at a concentration from 2.5% to 30% by weight of the composition.

5. The skin care composition according to any one of claims 1 to 4, wherein said composition further comprises a natural source of vitamin C, wherein said natural source of vitamin C is preferably selected from the group consisting of a plant, a herb, a leave, a vegetable or a fruit and mixtures thereof, more preferably selected from the group consisting of Kakadu Plum, Amazonian Camu Camu Berry, Sea Buckthorn, Rose Hip and mixtures thereof.

6. The skin care composition according to any one of the preceding claims 1 to 5, wherein said composition further comprises a natural source of vitamin A, a natural source of vitamin B, a natural source of tocotrienol, a natural source of vitamin E, or any mixture thereof.

7. The skin care composition according to any one of the preceding claims 1 to 6, wherein said skin care composition is a cream, preferably a face cream, body cream, or hand cream.

8. The skin care composition according to claim 7, wherein said composition further comprises white tea, preferably white matcha *(Thea sinensis* or varieties).

9. The skin care composition according to any one of claims 7 or 8, wherein said composition comprises coconut oil at a concentration from 50% to 70% by weight of the composition, preferably from 60% to 67%.

10. The skin care composition according to any one of claims 1 to 6, wherein said composition is a skin or face mask.

11. The skin care composition according to claim 10, wherein said composition further comprises green tea, preferably green matcha. *(Thea sinensis* or varieties).

12. The skin care composition according to any one of claims 10 or 11, wherein said composition comprises coconut oil at a concentration from 25% to 40% by weight of the composition, preferably from 30% to 35%, and wherein said composition optionally further comprises clay, preferably montmorillonite, or volcanic ash.

13. Use of a skin care composition according to any of the claims 1 to 12 in a prophylactic or remedial treatment of a skin exhibiting reduced elasticity by topical application of said composition to the skin.

14. A method for preparing a skin care composition according to any of claims 1 to 12 involving mixing the different components of said composition as set out in claims 1 to 12.
